# EUROPEAN PATENT SPECIFICATION

(11) **EP 1 462 711 B1**
(45) Date of publication and mention of the grant of the patent: **03.12.2014**
(21) Application number: 02796356.0
(22) Date of filing: 23.08.2002
(51) Int. Cl.: F21S 8/00, F21S 10/02, F21V 21/40, A61B 19/00, H05B 33/08, F21W 131/205, F21Y 101/02

(54) **COLOR TEMPERATURE-REGULABLE LED LIGHT**
FARBTEMPERATURREGELBARES LED-LICHT
ECLAIRAGE PAR DEL A TEMPERATURE DE COULEUR REGLABLE

(30) Priority: 23.08.2001 JP 2001252474
(43) Date of publication of application: 29.09.2004
(73) Proprietor: Okumura, Yukiyasu, Toyonaka-shi, Osaka 560-0002 (JP); Sierzputowski, Leszek P., 00-377 Warszawa (PL)
(72) Inventor: Okumura, Yukiyasu, Osaka 560-0002 (JP)
(74) Representative: Malewska, Ewa
(86) International application number: PCT/JP2002/008512
(87) International publication number: WO 2003/019072

(56) References cited:
- DE-U1- 20 007 134
- DE-U1- 29 620 583
- JP-A- 11 177 143
- JP-A- 11 243 232
- JP-A- 11 243 232
- JP-A- 61 032 483
- JP-A- 2000 164 931
- JP-A- 2000 340 840
- JP-A- 2001 190 499
- JP-B1- 45 016 391

## Description

### TECHNICAL FIELD

The present invention is directed to a LED lighting device, and more particularly to such a LED light suitable to a color temperature regulable LED shadowless light useful as a shadowless light preferably used for a surgical operation, a makeup light capable of reproducing a color temperature of a required location and enabling an decision of the most suitable makeup or not, and a living room light adapted for selection of a desired atmospheric color temperature.

### BACKGROUND ART

Nowadays, a halogen lamp is used as a surgical operation shadowless light. This halogen lamp is driven by a DC source having no flickers and so set as to render a color temperature of 4000°K optimal for an eye of the Japanese. However, since the halogen lamp generates a large quantity of heat, a shadowless light which employs the halogen lamp is provided with a cold mirror capable of reflecting more than at least 90 % of a visible ray as well as passing more than at least 90 % of a heat ray through itself to its backside, arranged behind the halogen lamp or provided with a cold filter capable of passing more than at least 90 % of a visible ray of a light radiated from the halogen lamp and reflected once to the backside as well as blocking the passing of almost of a heat ray so that the heat ray can be absorbed not to exert a thermic affect upon a surgical operation in the direction of a ray radiation.

However, since it is necessary to additionally provide such means for removing the thermic affect except the employing the halogen lamp, there are such problems that a cost is increased and a service time of the halogen lamp is short, for example about 2000 hours due to the thermic affect. Further, even though the heat ray is not radiated forwards, the thermic affect of the heat diverted to the backside is not removed yet. In addition, though it is a knowledge of the inventor of this invention that at least a suitable color temperature is to be set depending on an operation area (a brain surgery, a thoracic surgery, a celiac surgery, an orthopedics) for a surgical operation, the halogen lamp has only one kind of color temperature, which can not meet that request.

Accordingly, though the inventor of the present invention would like to propose a white LED which generates an extremely small amount of heat as well as has a long durable time (about 100,000 hours) without any flickers as a means for solving the former problem of the shadowless light for the surgical operation, the white LED needs the regulating of a wave length of a light radiated by the blue LED of a light source and the regulating of compositions of a fluorescent substance accompanied therewith for the regulating of the color temperature, which is the latter problem thereof and not easy.

In DE 200 07 134 U1 a lamp is discloses which comprises primary and secondary light sources of different color temperature characteristics. Individual continuous adjustment of color temperature and chromaticity of the light emitted by the lamp is possible by means of changing intensity of the secondary light source, the secondary light source emitting a color light or light of a different color temperature so that the resulting light has the characteristics differing from the characteristics of the primary light source. It has been suggested to use LED light source as the secondary light source.

Although the use of white LEDs instead of high or low pressure incandescent light sources is well known in the art and even though the use of color LEDs together with white light source in order to change the color temperature of the resulting light has been disclosed in DE 296 20 583 U1, it is believed that thus far the problem of precise and quantitative regulation of color temperature of light needed in lamps designed for surgical operations has not been effectively solved.

### DISCLOSURE OF THE INVENTION

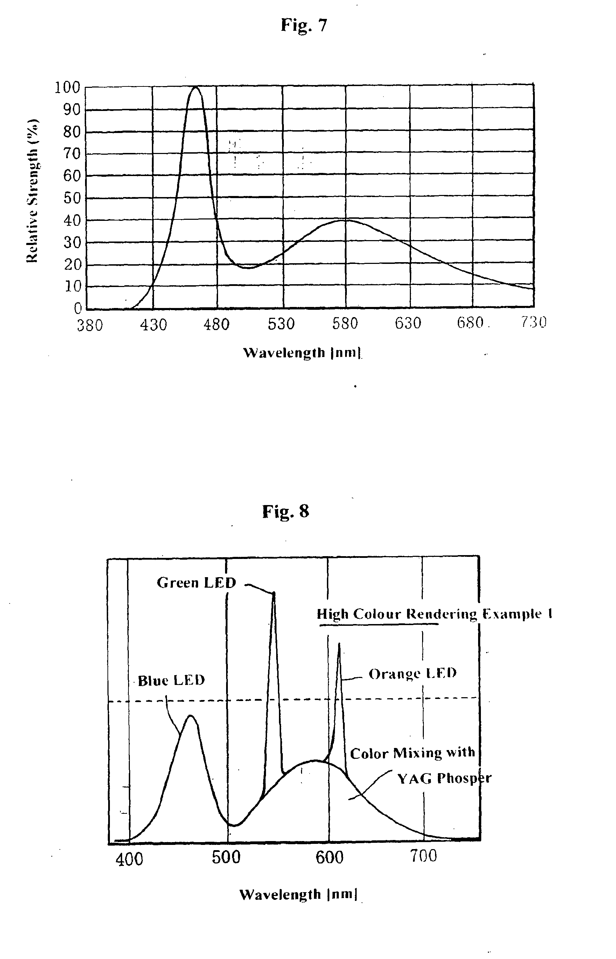

The inventor of the present invention has found for solving the above-mentioned latter problem by making an earnest effort that it is not only difficult to set a color temperature of the currently available white LED freely but also a color rendering property is poor, so that now the white LED is not suitable for a light of a surgical operation (Fig. 7 is a spectral graph of a white LED on the market comprising a LED having a light emitting layer composed of InGaN and a fluorescent substance composed of YAG:Ce). In order to solve such a problem, the inventor has found that it is preferable to perform a correction of a color temperature by considering not only a color temperature of the currently available white LED but also a spectrum distribution and that it is preferable to provide a correction
color LED having a peak wavelength within a specific wavelength region with respect to a white LED set to a specified color temperature so that not only a color temperature but also a color rendering can be corrected by the color mixing with that LED, by further making an earnest effort, and thus he has accomplished the present invention, which is defined in appended claims 1-3 and 12.

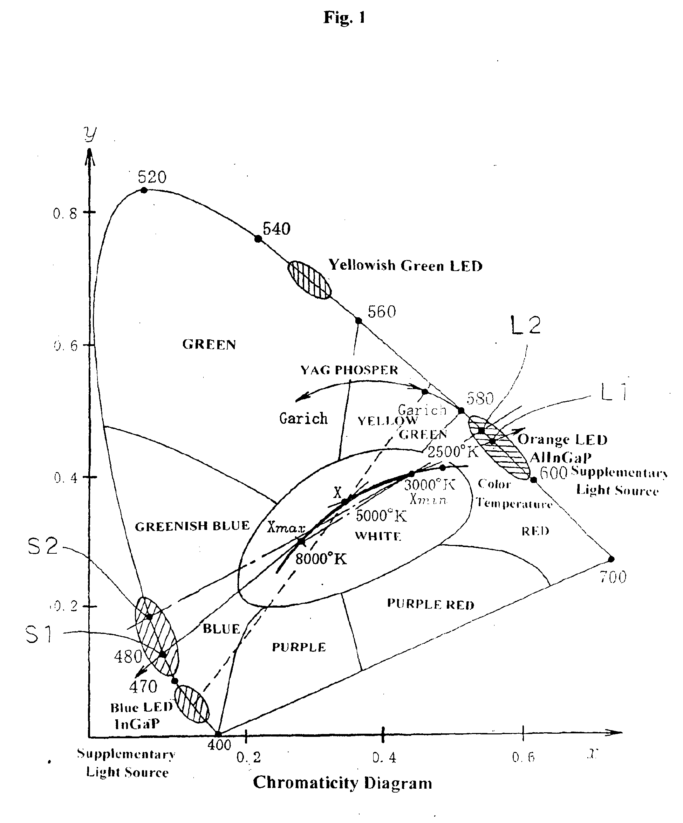

That is, the first invention is, as shown in Fig. 1, a LED lighting device which comprises (1) a white LED light source of which color temperature is set to a midpoint color temperature X, for example 5000°K between a predetermined minimum color temperature Xmin (K), for example 3000°K and a predetermined maximum color temperature Xmax (K), for example 8000°K, (2) a first correction LED light source a peak wavelength, for example 585 to 610 nm of an orange spectrum of which is positioned on or around a cross-point L1 (nm) defined by an intersection of an extension of a line segment between the minimum color temperature Xmin and the midpoint color temperature X with a longer wavelength side circumference of a chromaticity diagram, and (3) a second correction LED light source a peak wavelength, for example 470 to 490 nm of a blue spectrum of which is positioned on or around a cross-point S1 (nm) defined by an intersection of an extension of a line segment between the maximum color temperature Xmax and the midpoint color temperature X with a shorter wavelength side circumference of the chromaticity diagram, wherein a resultant color temperature from a combination of the white LED light source with the first or the second correction LED light source can be adjusted depending on a mixing ratio of spectra intensities of the two sources in such a manner as to get a color approaching the minimum color temperature or the maximum color temperature, for example the resultant color temperature belonging to the white LED light source can be adjusted by the color mixing with the first correction LED light source so as to get a desired color approaching the minimum color temperature or with the second correction LED light source so as to get a desired color approaching the maximum color temperature. Incidentally, in the present invention, a color temperature means a color temperature assumed on a black body radiation line defined on a chromaticity diagram and a color temperature approximated thereto.

The second invention is a LED lighting device which is contemplated to set the white LED light source to a minimum color temperature Xlow (K) and comprises (1) the white LED source, and (2) a third correction LED source a peak wavelength of a spectrum of which is positioned on or around a cross-point S2 (nm) defined by an intersection of an extension of a line segment between the minimum color temperature Xlow and at least one of the color temperatures X with a chromaticity diagram on a shorter wavelength side in the chromaticity diagram, and wherein a resultant color temperature from a combination of the white LED light source with the third correction LED light source can be adjusted depending on a mixing ratio of spectral intensities of the two sources in such a manner as to get a color approaching the maximum color temperature.

The third invention is a LED lighting device which is contemplated to set the white LED light source to a maximum color temperature Xhigh (K) and comprises (1) the white LED light source, and (2) a fourth correction LED light source a peak wavelength of a spectrum of which is positioned on or around a cross-point L2 (nm) defined by an intersection of an extension of a line segment between the maximum color temperature Xhigh and at least one of the color temperatures X with a chromaticity diagram on a longer wavelength side in the chromaticity diagram, and wherein a resultant color temperature from a combination of the white LED light source with the fourth correction LED light source can be adjusted depending on a mixing ratio of spectral intensities of the two sources in such a manner as to get a color approaching the minimum color temperature.

### BRIEF DESCRIPTION OF THE INVENTION

Fig. 1 is a graph showing a setting method and an adjusting method of a color temperature on a chromaticity diagram.
Fig. 2 is a schematic view showing a method for utilizing an auxiliary light source in a surgical operating table.
Fig. 3 is a schematic view showing a method for utilizing an auxiliary light source in a shadowless light.
Fig. 4 is a plan view showing LED locations in one embodiment.
Fig. 5 is a plan view showing an arrangement of a LED head in one embodiment.
Fig. 6 is a sectional view showing a mounted condition of a LED head in one embodiment.
Fig. 7 is a graph showing typical spectra of a white LED on the market.
Fig. 8 is a graph showing spectra of a first example of a high color rendering property which can be accomplished by a LED light according to the present invention.
Fig. 9 is a graph showing spectra of a second example of a high color rendering property which can be accomplished by a LED light according to the present invention.
Fig. 10(a) is a view showing a method for uniformly mixing of LED lights by using a spherical reflecting mirror and obtaining a predetermined illumination aperture and Fig. 10(b) is a perspective view of a concrete example of a white LED for use in the LED light in an enlarged scale.
Fig. 11 (a) is a sectional view showing a conventional improved LED lighting device applied as a down-light and Fig.11 (b) is a schematic sectional view showing a LED lighting device according to the present invention applied as a down-light.
Fig. 12 is a perspective view for explaining a control method of a LED lighting device according to the present invention applied to a control system of a conventional living room light.
Fig. 13(a) is a graph showing one relationship between a color temperature and an illumination during a certain time zone and Fig. 13(b) is a graph showing the other relationship therebetween.

### DETAILED DESCRIPTION OF THE PREFERRED EMBODIMENT

A white LED source according to the present invention is an assembly of a LED an emission peak wavelength of which is adjusted to a blue region of 400 to 490 nm, preferably 430 to 470 nm and a YAG fluorescent substance which is excited by the LED and a fluorescent peak wavelength of which is adjusted to a yellow region of preferably 530 to 580 nm (for example, refer to Fig. 7). A wavelength close to a violet region of 400 nm around may be used for the LED as required so as to radiate a white light by selecting a suitable fluorescent substance.

For realizing the setting of a color temperature of the white LED light source, as shown in Fig. 1, first a peak wavelength point of the blue LED is connected with a required color temperature in the graph to define a line segment, and then a peak wavelength of the fluorescent spectrum is decided by an intersection of an extension of the line segment with a chromaticity diagram on a longer wavelength side in the diagram as well as a composition of a fluorescent substance is decided. Resultantly, the setting of that color temperature is realized by combination of both those decisions. The white LED may be designed by setting a central color temperature on the chromaticity diagram and then by considering a peak wavelength of the LED decided by an intersection of an extension from the central color temperature with the chromaticity diagram on a longer wavelength side in the diagram and a peak wavelength of the fluorescent substance decided by an intersection of the extension with the chromaticity diagram.

As a LED adapted to adjusting a resultant color temperature from the white LED light source so as to approach the maximum color temperature depending on a mixing ratio with the white LED light source may be selected a blue LED an emission peak wavelength of which has been adjusted to a blue region of preferably 430 to 470 nm.

As a LED adapted to adjust a resultant color temperature from the white LED light source so as to approach the minimum color temperature depending on a mixing ratio with the white LED light source may be selected an orange LED an emission peak wavelength of which has been adjusted to an orange region of preferably 585 to 630 nm.

Thereby, a color temperature can be adjusted to a region of 3000 to 8000°K as a whole depending on a color mixing ratio of the main light source LED being the white LED and the auxiliary light source LED being the blue LED and/or the orange LED.

Color mixture in the main light source LED is determined by a ratio of an intensity (or an area) of a transmission spectrum through a YAG fluorescent substance layer and an intensity (or an area) of an emission spectrum from a YAG fluorescent substance layer of the light source LED. When the ratio is set in 2 : 1 to 1 : 4, a various kinds of spectra can be obtained. For example, a spectrum of the white LED on the market, manufactured by NICHIA Chemical Co. LTD., is shown in Fig. 7. When an amount of fluorescent substance is increased with respect to this spectrum, spectra of the blue region are decreased, so that somewhat yellow color mixture can be obtained. To the contrary, when the spectra of the blue region of that LED are increased, somewhat blue color mixture can be obtained. Therefore, a person having ordinary skill in the art can set a color temperature in consideration of a spectral distribution.

### [Color Temperature Adjustment Without Use of Correction LED]

In an alternative arrangement of a color temperature adjustment employing a color correction LED, one kind of or two or more kinds of auxiliary LED an emission peak wavelength of which has been adjusted to an invisible region are arranged in a group of main light source LEDs suitably in a dispersed condition, and a fluorescent substance adapted to be excited by the auxiliary LED and to radiate a fluorescence ray having a peak wavelength in a blue region of 430 to 490 nm or a fluorescence ray having a peak wavelength in an orange through red region of 590 to 700 nm is contained in a filter which covers the LEDs. Thereby, the color temperature having a filter transmission wavelength can be adjusted within a range of 3000 to 8000 °K by mixing emission spectra from the main light source LEDs and an emission fluorescent spectrum from the filter. Further, when a color temperature converting filter is provided so as to cover the white LEDs as the main light source, a color temperature having of an emission wavelength can be adjusted within a range of 3000 to 8000 °K.

### [Alternative Method Without Use of White LED]

Besides the above-mentioned white LED, the following method can provide a white light source.

In a first alternative arrangement, a first blue LED having an emission peak wavelength adjusted to a blue region of 430 to 470 nm, a second LED having a peak wavelength adjusted to a yellowish green region of 530 to 570 nm and a third LED having a peak wavelength adjusted to an orange region of 585 to 630 nm, preferably 585 to 600 nm are arranged so as to provide a white color as a whole by mixture of emission spectra from them. In this case, when the first and the third LEDs are dispersed in a group of the second LEDs arranged as a main component, by adjusting relative intensities of radiation of lights from the first LED and the third LED relative to the second LED, a mixture color temperature may be adjusted within a range of 3000 to 8000 °K as a whole. It is preferable to arrange the first and the third LEDs in a group of the second LEDs so as to obtain a ratio of 1 ± 1 :
3 : 2 ± 1 as a whole. In spite of being variable depending on their half-value ranges, when ranges of wavelength regions of respective LEDs are comparatively narrow, the LEDs may preferably arranged in such a dispersed condition as for the light emission peak wavelengths of the respective regions to take a ratio of 1 : 3 : 2.

In a second alternative arrangement, on one hand, auxiliary LEDs having an emission peak wavelength adjusted to an invisible region of 530 to 570 nm are arranged suitably in a dispersed condition into a group of main light source LEDs having an emission peak wavelength adjusted to a blue region of 430 to 470 nm, and on the other hand, a YAG fluorescent substance adapted to be excited by the main light source LED and to radiate a fluorescent spectrum having a peak wavelength in a yellowish green region of 530 to 570 nm, a fluorescent substance adapted to be excited by the auxiliary LED and to radiate a fluorescent spectrum having a peak wavelength in a blue region of 430 to 490 nm and a fluorescent substance adapted to radiate a fluorescent spectrum having a peak wavelength in an orange through red region of 590 to 700 nm are contained in a filter which covers the LEDs. Therefore, by adjusting intensities of the main light source LEDs and intensities of the auxiliary LEDs, the color temperature having a wavelength of a filter transmission light can be adjusted to a range of 3000 to 8000 °K by the filter.

### [Spectrum Having High Color Rendering Property]

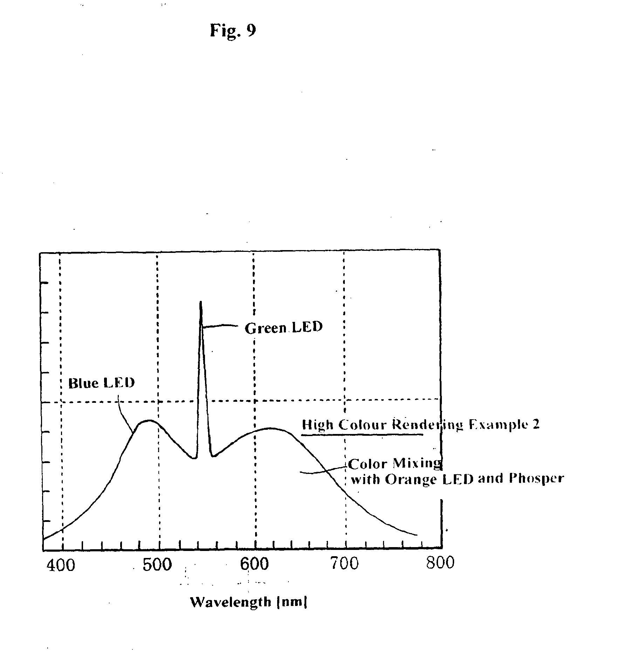

For enhancing a color rendering property, it is preferable to form spectra as shown in Fig. 8 and Fig. 9. The spectra shown in Fig. 8 and Fig. 9 can be formed by not only complementing the orange LED in the white LED light source but also providing a complementary by using the green LED. A difference between the spectrum forming methods of Fig. 8 and Fig. 9 is based on a difference of an amount of YAG fluorescent substance to be used with respect to the blue LED and a difference of an amount of complementary in the orange LED. When the complementary is provided at least by the green LED, the color rendering property can be enhanced. But, it is preferable to change the color temperature by using the blue LED light source or the orange LED light source as the auxiliary light source for the white LED because the color temperature can be changed with enhancing the color rendering property rather than damaging it.

### [Application Method to Existing Equipment]

When the LED light is directly applied to an AC power source of 100 V or the likes similarly to an ordinary electric bulb lamp, it is necessary to provide the LED light with a mouth-piece to be connected to the AC power source, a transformer connected to the mouth-piece to transform an AC voltage and a light regulator for setting and supplying a predetermined DC voltage to the main light source LED and the auxiliary LED respectively.

When it is applied to a DC power source for a shadowless light, it is necessarily provided with a mouth-piece to be connected to the DC power source of 24 V and a light regulator for setting and supplying a predetermined DC voltage to the main light source LED and the auxiliary LED respectively.

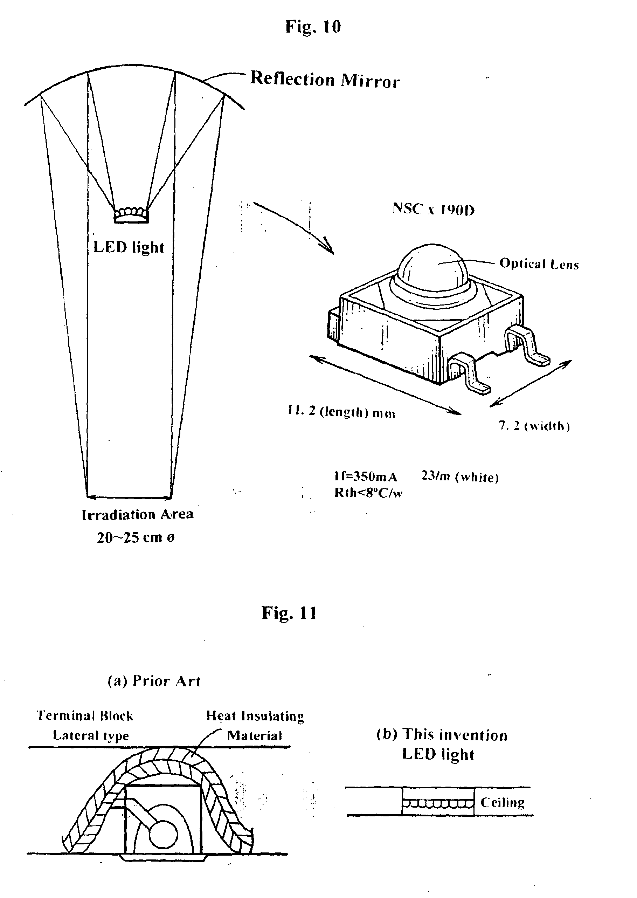

It is preferable to use the LED lighting device of the present invention as a down-light in a living room. As shown in Fig. 11 (a), even though a conventional down-light is disposed in a oblique manner, it is essential to thermally shield it by a heat insulating material. In contrast, when the LED lighting device which scarcely generates a heat is used, it can be installed in a flat manner, for example so as to be buried in a ceiling panel board. When it is applied to a remote control system for existing living room lights, it can be set as follows.

In the remote control system for existing living room lights, for example four kinds of lighting scenes such as a happy home scene, a mealtime scene, a relaxation scene and an AV scene are selected and three kinds of lighting conditions such as a full lighting, a half lighting and a light-out of each lighting device such as a light I-1 to a light I-4 and a light II- 1 to a light II-4 can be selected corresponding to each scene. When the lighting device according to the present invention is applied to the respective lights I-1 to I-4 and II-1 to II-4, a color temperature suitable for each scene is set in consideration of a season and a time zone such as a morning, a daytime and a night so that a lighting schedule can be decided in combination of those conditions.

Accordingly, superior lighting effects which haven't ever been experienced can be realized readily and enjoyed by manipulating a remote control switch.

Incidentally, the lights I-1 to I-4 are living LED lights, the lights II-1, II-2 and II-4 are LED down-lights and the light II-3 is a sealing LED light.

### [Lighting Schedule]

When converting a color temperature of a conventional lighting device, first an incandescent lamp is turned on and then a fluorescent lamp is turned on so as to measure a rising of a color temperature, and finally the incandescent lamp is turned off so as to obtain a maximum color temperature. In this method, however, it is difficult to set a continuous color temperature and thus it is impossible to set the color temperature freely. According to the present invention, since the LED lighting device which allows the free conversion of the color temperature and the illumination can be utilized, it is unnecessary to change-over the incandescent lamp to the fluorescent lamp, it is possible to set the color temperature and the illumination freely and the light regulation can be controlled very readily.

By making use of a remote controller with a built-in microcomputer and sensors, the following controlling can be readily carried out on hand.
1) Setting of the time of rising and the bedtime;
2) Setting of the favorite illumination and color temperature;
3) Indicating of the clock;
4) Setting of the lighting on and the lighting off at favorite times;
5) Setting of the controlling of the light regulation according to the stored one during 24 hours.

For example, in this method, the LED lighting device is turned on 30 mins before a waking time to start the light regulation. The lighting device increases an intensity of its light gradually so as to get brighter with a slow rhythm like the rising sun to stimulate a brain of a user by the light passing through eyelids and to conduct the user into a waking condition gradually. When it has reached the time of rising, it becomes possible to make the user wake-up with a refreshed feeling by the bright light having its color temperature further increased and its larger illumination. When it has been evening, it becomes possible to produce a room having a staid atmosphere and to regain the presence of mind by the low color temperature.

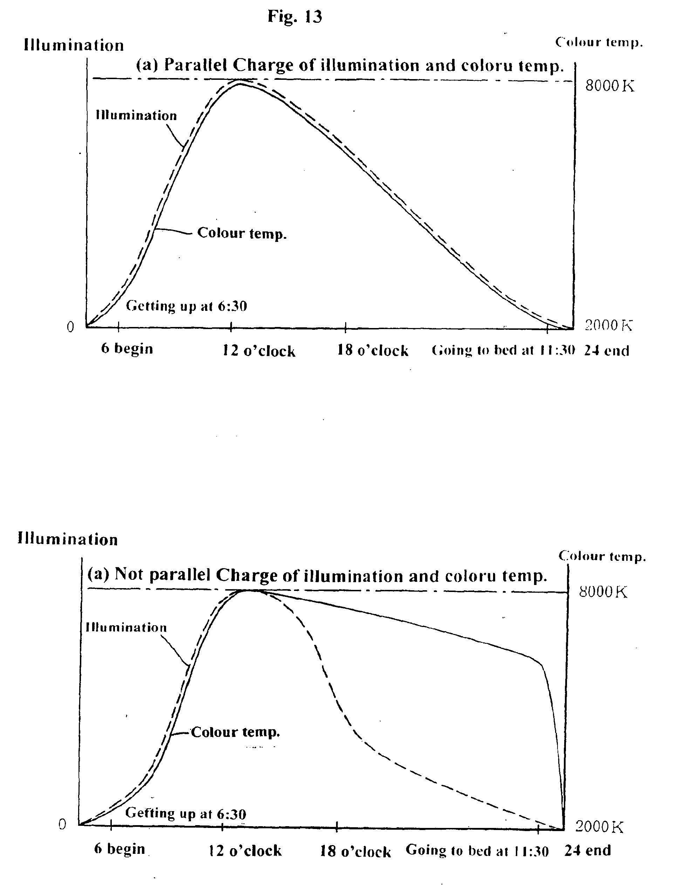

These functions of this lighting device can be used in lights for a show window, an advertisement, a clothes-selling floor space, a cosmetics-selling floor space, and so on to produce simulated lights in different time zones of a day for enabling a person to select fitted clothes, make-ups and so on in each scene. In a case of a surgical operation, it is possible to automatically or optionally select the color temperature and the illumination suitable for each section of an operational preparation time, an initial stage of the operation, a middle stage thereof, an end stage thereof and a break time. Fig. 13 shows a relation between the color temperature and the illumination along a time zone of a day.

### [Light Concentration and Dispersion Mixing of LED Light]

A base plate to which the main light source LED and the auxiliary LED are attached may be provided with a spherical reflective mirror for concentrating a light moderately. In addition, lenses installed in a front may be so arranged as to mix lights well radiated from various kinds of LEDs as required. With such an arrangement, the lights from the various kinds of LEDs can be mixed evenly. Especially, in a shadowless light for a surgical operation, since an operation area needs a light of high illumination, for example 100,000 luxes, a light from the LED is reflected once by the spherical reflective mirror as shown in Fig. 10 so as to be adjusted to have a desired irradiation aperture. When using the white LED of NSCx 190D developed by Nichia Chemical Co. LTD., it is possible to obtain the illumination of 100,000 luxes by using about 140 pieces of LEDs for the irradiation aperture having a diameter of 20 cm or about 210 pieces of LEDs for the irradiation aperture having a diameter of 25 cm. Since the LED lighting device scarcely generates a heat and has a long durable time, it presents the optimal condition for a surgical operation.

### [LED Auxiliary Light Source]

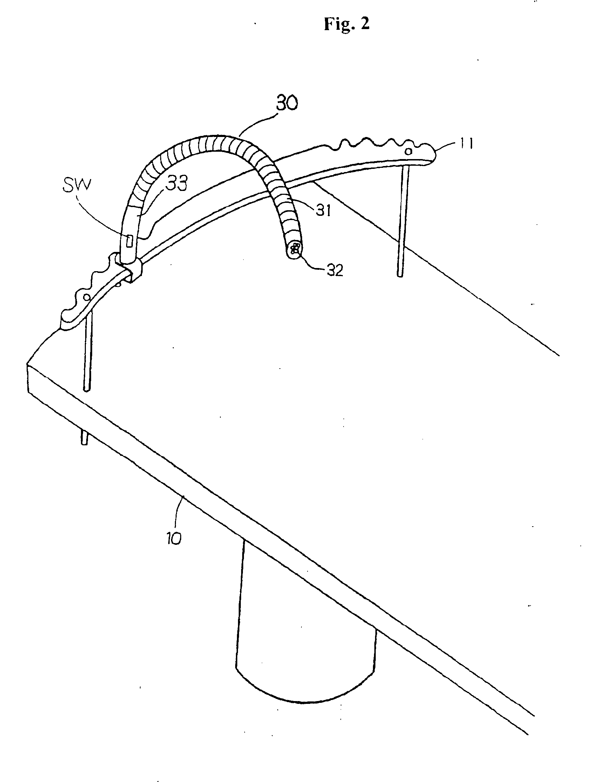

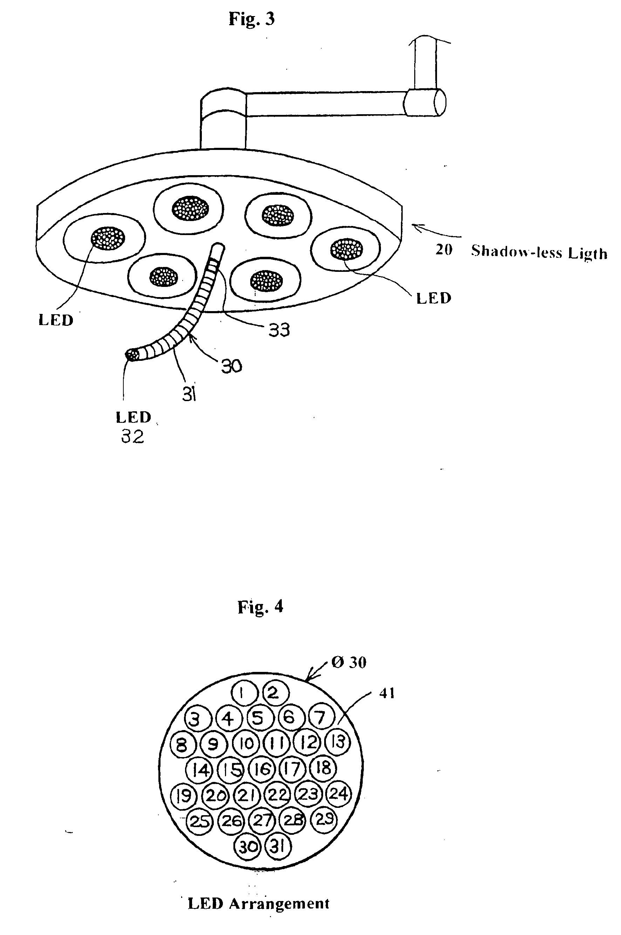

A shadowless lighting device is preferably installed as an operating light within an operating room. Therefore, it is preferable that an irradiating direction of an auxiliary light source can be readily changed to assist the shadowless lighting device. When employing the above-mentioned arrangement of the white LED and so on, it becomes possible to provide an auxiliary light source an irradiating direction of which can be readily. For example, as shown in Fig. 2, the LED auxiliary light source is attached to an existing bar 11 mounted to an operating table 10 at its one end, or as shown in Fig. 3, it is attached to a handle portion21 projecting downwardly from a central portion of an existing shadowless lighting device 20.

A LED auxiliary light source device 30 comprises a LED light source 32 including a number of LEDs buried in a leading end portion of a flexible tube 31, and a DC electric power source 33 of about 4 V having batteries serially connected to one another in a base end portion thereof and connected to the LED light source 32. When being attached to the existing bar 11 of the operating table or the handle portion 21 of the shadowless lighting device, a conventional fixing mechanism such as of a screwed-in type, a clamping type or the like may be employed. By employing such an auxiliary light source, it becomes possible to assist the main light source of the shadowless lighting device, particularly so as to increase an illumination in an operating area. Further, since a heat is scarcely generated from the auxiliary light source, there are no obstacles to an operation by an operating surgeon.

### Embodiment 1

The white LED (W) having a color temperature of about 5000°K is formed by combining a blue LED having its emission peak wavelength adjusted to a blue region of about 450 to 460 nm with a YAG fluorescent substance adapted to be excited by the blue LED and having its emission peak wavelength adjusted to a yellowish green region of about 570 nm.

Next, a first correction light source comprising a blue LED (B) with an active layer of InGaN having its emission peak wavelength adjusted to a blue region of about 470 to 480 nm and a second correction light source comprising an orange LED (Am) with an active layer of AllnGaP having its emission peak wavelength adjusted to an orange region of about 590 nm are provided.

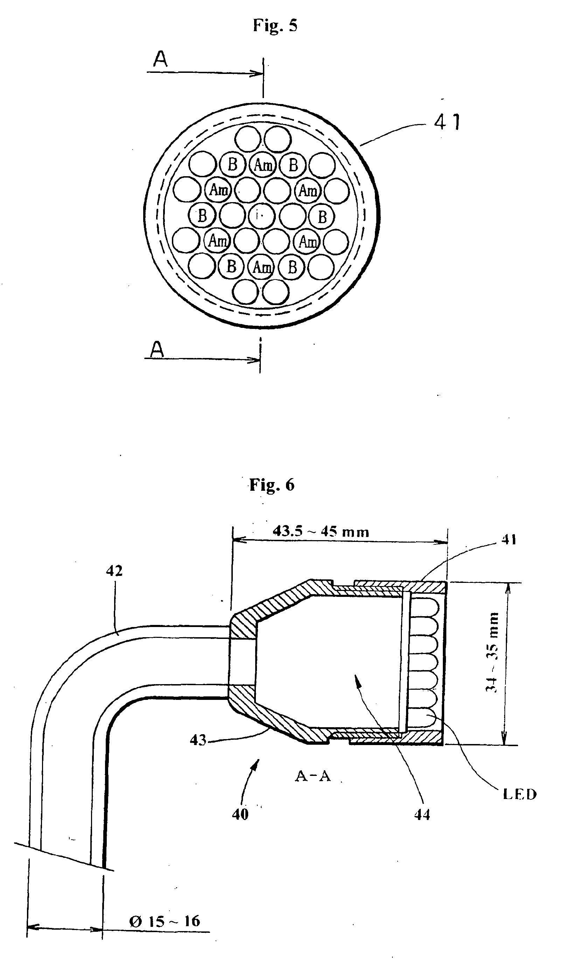

As shown in Fig. 4, 31 pieces of those LEDs are arranged on a base board. As shown in Fig. 5, the arrangement is composed of a center white LED (W) (16), a first circle including 6 pieces of white LEDs (W) (10, 11, 17, 22, 21, 15), a third circle including 12 pieces of white LEDs (W) (1,2,7,13,24,29,31,30,25,19,8,3), and a second circle including 6 pieces of blue LEDs (B) (4,6,18,28,26,14) and 6 pieces of orange LEDs (Am) (5,12,23,27,20,9) arranged alternately one another.

As shown in Fig. 6, in this LED lighting device 41, 6 pieces of blue LEDs (B) and 6 pieces of orange LEDs (Am) are connected to a DC power source through a fixed or a variable resistance 44 so that their illuminations can be adjusted, while the white LEDs are directly connected to the DC power source (3.6 to 4.5 V). A connecting method may be changed depending on a voltage of the power source. This LED lighting device 41 is attached to a leading end head 43 of a flexible arm 42 to form a LED shadowless light a color temperature of which can be adjusted. When being used in place of an existing shadowless halogen lamp, the LED lighting device 41 is mounted through a mouth-piece attached to a base end of the leading end head 43.

When only the white LEDs are turned on, a white light having a color temperature of 5000°K can be obtained, but its color rendering property is poor. Wherein, when three to six pieces of LEDs are also turned on, the color rendering property is improved as well as the color temperature is lowered to 3000 to 6000°K. On the other hand, when all of the white LEDs and three to six pieces of blue LEDs are turned on, the color temperature is raised to 8000°K or more, but the color rendering property is not improved. Thus, additionally two to three pieces of orange LEDs are turned on, the color temperature is lowered to 8000 to 6000°K around, but the color rendering property is improved.

In this way, by mixing a color of the main light source LED with a color of the auxiliary light source LED, a light-emission color temperature can be adjusted within a range of 3000 to 8000°K as a whole. Since the orange LED serves to improve the color rendering property and to lower the color temperature and the blue LED serves to raise the color temperature but doesn't serve to improve the color rendering property, both the color temperature and the color rendering property can be adjusted by balancing the lighting of the orange LEDs with the lighting of the blue LEDs.

In the above-mentioned embodiment, though the main light source is set to a middle color temperature and the color temperature is adjusted toward a lower temperature or toward a higher temperature by using the auxiliary light source, the color temperature of the main light source may be set to about 8000°K and the color temperature may be adjusted toward the lower temperature by using the orange LED of about 590 nm as the auxiliary light source. In addition, the color temperature of the main light source may be set to about 3000°K and the color temperature may be adjusted toward the higher temperature by using the blue LED of about 480 nm as the auxiliary light source. In this case, the orange color may be optionally mixed depending on a degree of the color rendering effect.

Following programs show procedures for adjusting a color temperature depending on an operation area.

### [First Program]

Brain surgery 8000°K, Thoracic surgery 6000°K, Celiac surgery 5000°K, Orthopedic surgery 4000°K
   (Correction color: Blue on-off)← White →(correction color: Orange: on-off) [Second Program]
Brain surgery 8000°K, Thoracic surgery 6000°K, Celiac surgery 5000°K, Orthopedic surgery 4000°K
   (Corr. color: Blue Strong-Weak)← White →(Corr. color: Orange: Strong-Weak) Initial setting: Blue (Strength 1) Yellow (Strength 3) Orange (Strength 2)

The first program shows that the white LED (5000°K) is used as the main light source and the blue LED and the orange LED are used as the auxiliary light source so as to correct the color temperature.

The second program shows that the white light source is produced by mixing colors emitted from the blue LED, the yellow LED and the orange LED and the color temperature is corrected by adjusting the strength and weakness of the blue or the orange.

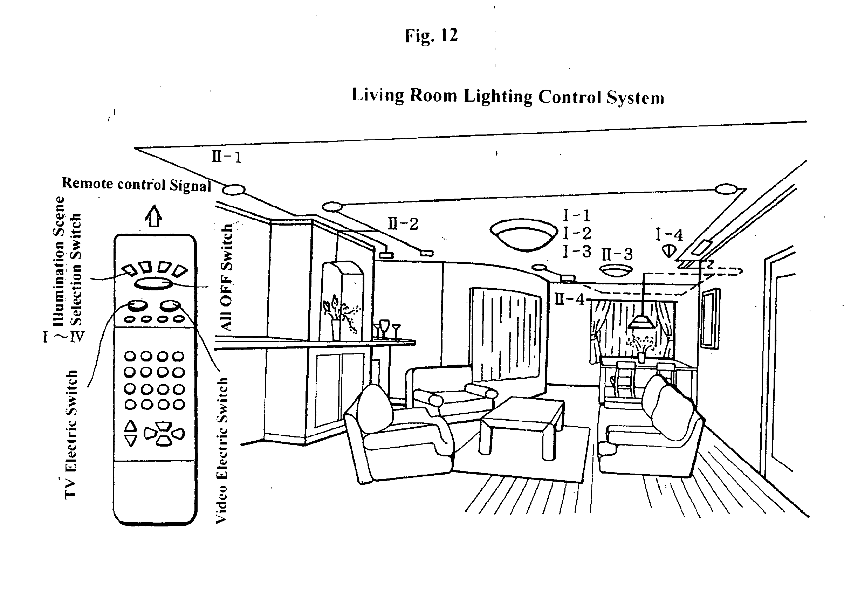

Incidentally, since a color having a high color saturation can be obtained by removing a ray of 580 nm around from a visible ray, a spectrum which rises from a blue region of 450 nm to a yellow region of 550 nm, then lowers once and rises again from a neighborhood beyond 600 nm can be formed. The setting of the color temperature for the shadowless lighting device uses the remote control system as shown in Fig. 12, so that when a number of scenes have been previously set, a desired color temperature and a desired color rendering property can be realized simultaneously in each scene.

According to the present invention, it becomes possible to adjust a white light so as to have a different color temperature by correcting the white light emitted from the white LED or the LED color mixture.

Therefore, when being used in a shadowless lighting device, it becomes possible to set a color temperature depending on an operation area, for example to 8000°K for brain surgery, to 6000°K for thoracic surgery, to 5000°K for celiac surgery 5000°K and to 4000°K for orthopedic surgery and also to obtain a superior color rendering property because an orange LED is used for correcting a color. Accordingly, a surgeon can carry out a surgical operation with a favorite color temperature and moreover in a comfortable operational environment because the LED scarcely generates a heat.

Since the LED lighting device capable of adjusting the color temperature is used, it is also possible to produce different atmospheres by changing a color temperature of a light in a room. For example, a correlation color temperature of 6700 °K produces a cool color (a cool atmosphere, a refreshing atmosphere), a correlation color temperature of 5000°K produces a natural color (a natural atmosphere), a correlation color temperature of 3000°K produces a warm color (a calm atmosphere).

Further, it is recommendable to use the present invention as a make-up LED light the setting of a color temperature of which plays an important role. Also in this case, as the make-up light can be used such a system that desired conditions are framed as respective corresponding scenes as well as a quantity of light in an auxiliary light source relative to a main light source is adjusted manipulatively, preferably automatically by a switch or a remote control so that a color temperature of a required location can be selected.

## Claims

1. A LED lighting device comprising (1) a white LED light source a color temperature of a light emitted from which is adjusted to a midpoint color temperature X between a predetermined minimum color temperature Xmin (K) and a predetermined maximum color temperature (K) on a black body radiation line, (2) a first correction LED light source having an emission spectrum a peak wavelength of which is positioned on or around a cross-point L1 (nm) defined by an intersection of an extension of a line segment between the minimum color temperature Xmin and the midpoint color temperature X with a longer wavelength side circumference of a chromaticity diagram, and (3) a second correction LED light source having an emission spectrum a peak wavelength of which is positioned on or around a cross-point S1 (nm) defined by an intersection of an extension of a line segment between the maximum color temperature Xmax and the midpoint color temperature X with a shorter wavelength side circumference of the chromaticity diagram, wherein a resultant color temperature from a combination of the white LED light source with the first or the second correction LED light source can be adjusted depending on a mixing ratio of spectra intensities of the two sources in such a manner as to get a color approaching the minimum color temperature or the maximum color temperature, and wherein (1) the white LED light source is composed in combination of a LED an emission peak wavelength of which is adjusted to a blue region of 430 to 470 nm and a fluorescent substance which is excited by the LED and a fluorescent peak wavelength of which is adjusted to a yellow region of 530 to 570 nm, and wherein (2) an auxiliary LED light source comprising the first and second correction LED light sources comprises a blue LED an emission peak wavelength of which is adjusted to a blue region of 430 to 470 nm and an orange LED a peak wavelength of which is adjusted to an orange region of 590 to 630 nm, and wherein a resultant color temperature from either of the white LED light source and the auxiliary LED light source or a combination of both the light sources can be adjusted within a range of 3000 to 8000 °K as a whole.

2. A LED lighting device comprising (1) a white LED light source which is adjusted to a predetermined minimum color temperature Xlow (K) on a black body radiation line, and (2) a third correction LED source having an emission spectrum a peak wavelength of which is positioned on or around a cross-point S2 (nm) defined by an intersection of an extension of a line segment between the minimum color temperature Xlow and at least one of the color temperatures X selected on a black body radiation line on the higher temperature side with a shorter wavelength side circumference of the chromaticity diagram, wherein a resultant color temperature from a combination of the white LED light source with the third correction LED light source can be adjusted depending on a mixing ratio of spectral intensities of the two sources in such a manner as to get a color approaching a maximum color temperature, and wherein (1) the white LED light source is composed in combination of a LED an emission peak wavelength of which is adjusted to a blue region of 430 to 470 nm and a fluorescent substance which is excited by the LED and a fluorescent peak wavelength of which is adjusted to a yellow region of 530 to 570 nm, and wherein (2) an auxiliary light LED source comprising the third correction LED source comprises a blue LED an emission peak wavelength of which is adjusted to a blue region of 430 to 470 nm and an orange LED a peak wavelength of which is adjusted to an orange region of 590 to 630 nm, and wherein a resultant color temperature from either of the white LED light source and the auxiliary LED light source or a combination of both the light sources can be adjusted within a range of 3000 to 8000 °K as a whole.

3. A LED lighting device comprising (1) a white LED light source which is adjusted to a predetermined maximum color temperature Xhigh (K) on a black body radiation line, and (2) a fourth correction LED source having an emission spectrum a peak wavelength of which is positioned on or around a cross-point L2 (nm) defined by an intersection of an extension of a line segment between the maximum color temperature Xhigh and at least one of the color temperatures X selected on a black body radiation line on the lower temperature side with a longer wavelength side circumference of the chromaticity diagram, wherein a resultant color temperature from a combination of the white LED light source with the fourth correction LED light source can be adjusted depending on a mixing ratio of spectral intensities of the two sources in such a manner as to get a color approaching a minimum color temperature, and wherein (1) the white LED light source is composed in combination of a LED an emission peak wavelength of which is adjusted to a blue region of 430 to 470 nm and a fluorescent substance which is excited by the LED and a fluorescent peak wavelength of which is adjusted to a yellow region of 530 to 570 nm, and wherein (2) an auxiliary LED light source comprising the fourth correction LED light source comprises a blue LED an emission peak wavelength of which is adjusted to a blue region of 430 to 470 nm and an orange LED a peak wavelength of which is adjusted to an orange region of 590 to 630 nm, and wherein a resultant color temperature from either of the white LED light source and the auxiliary LED light source or a combination of both the light sources can be adjusted within a range of 3000 to 8000 °K as a whole.

4. A LED lighting device according to any one of claims 1 to 3, wherein the white LED light source has a ratio between an intensity of its YAG fluorescent layer transmitting spectrum and an intensity of its YAG fluorescent layer emitting spectrum set to 2 : 1 to 1 : 4,

5. A LED lighting device as set forth in any one of claims 1 to 4, wherein further comprising a mouth-piece to be connected to an AC power source, a transformer for transforming the AC power source, and a light regulator to be connected to the transformer and to set and supply a predetermined DC voltage to the main light source LED and the auxiliary LED respectively.

6. A LED lighting device as set forth in any one of claims 1 to 4, wherein further comprising a mouth-piece to be connected to a DC power source for a shadowless lighting device, a transformer, and a light regulator to be connected to the transformer and to set and supply a predetermined DC voltage to the main light source LED and the auxiliary LED respectively.

7. A LED lighting device as set forth in any one of claims 1 to 4, wherein a base board to which the white LED light source and the auxiliary LED light source are attached is provided in its front with a spherical reflective mirror to obtain a white light without color unevenness by color mixture of the reflected lights.

8. A LED lighting device as set forth in any one of claims 1 to 4, wherein further comprising a system for adjusting a quantity of light from the auxiliary LED light source with respect to the white LED light source by manipulating a switch or a remote control for that adjustment.

9. A LED lighting device as set forth in claim 8, wherein the system for adjusting a quantity of light from the auxiliary LED light source with respect to the white LED light source by manipulating a switch or a remote control for that adjustment is configured such that an optimal color temperature for an operation area, can be selected.

10. A LED lighting device as set forth in claim 8, wherein the system for adjusting a quantity of light from the auxiliary LED light source with respect to the white LED light source by manipulating a switch or a remote control for that adjustment is configured such that a color temperature for a desired area can be selected.

11. A LED lighting device as set forth in claim 8, wherein the system for adjusting a quantity of light from the auxiliary LED light source with respect to the white LED light source by manipulating a switch or a remote control for that adjustment is configured such that a desired atmospheric color temperature can be selected

12. A method for regulating a color temperature of a light emitted by a LED lighting device wherein
(a) a white LED light source a color temperature of a light emitted from which is adjusted to a midpoint color temperature X between a predetermined minimum color temperature Xmin (K) and a predetermined maximum color temperature (K) on a black body radiation line, (2) a first correction LED light source having an emission spectrum a peak wavelength of which is positioned on or around a cross-point L1 (nm) defined by an intersection of an extension of a line segment between the minimum color temperature Xmin and the midpoint color temperature X with a longer wavelength side circumference of a chromaticity diagram, and (3) a second correction LED light source having an emission spectrum a peak wavelength of which is positioned on or around a cross-point S1 (nm) defined by an intersection of an extension of a line segment between the maximum color temperature Xmax and the midpoint color temperature X with a shorter wavelength side circumference of the chromaticity diagram is provided; or
(b) a white LED light source which is adjusted to a predetermined minimum color temperature Xlow (K) on a black body radiation line, and (2) a third correction LED source having an emission spectrum a peak wavelength of which is positioned on or around a cross-point S2 (nm) defined by an intersection of an extension of a line segment between the minimum color temperature Xlow and at least one of the color temperatures X selected on a black body radiation line on the higher temperature side with a shorter wavelength side circumference of the chromaticity diagram is provided; or else
(c) a white LED light source which is adjusted to a predetermined maximum color temperature Xhigh (K) on a black body radiation line, and (2) a fourth correction LED source having an emission spectrum a peak wavelength of which is positioned on or around a cross-point L2 (nm) defined by an intersection of an extension of a line segment between the maximum color temperature Xhigh and at least one of the color temperatures X selected on a black body radiation line on the lower temperature side with a longer wavelength side circumference of the chromaticity diagram is provided; and
the resultant color temperature from a combination of the white LED light source with the correction LED light source is adjusted by means of a mixing ratio of spectra intensities of the two sources In such a manner as to get a color approaching the minimum color temperature or the maximum color temperature.

13. A method according to claim 12 wherein the LED lighting device is according to any one of claims 1-11

14. A method according to claim 13, wherein the main light source LED has a ratio between an intensity of its YAG fluorescent layer transmitting spectrum and an intensity of its YAG fluorescent layer emitting spectrum set to 2:1 to 1:4.

## Patentansprüche

1. LED-Beleuchtungsanlage, bestehend aus (1) einer weißen LED-Lichtquelle, in welcher die Farbtemperatur des von dieser Quelle emittierten Lichts an die Farbtemperatur des mittleren Punktes X zwischen der zuvor festgelegten minimalen Farbtemperatur Xmin (K) und der zuvor festgelegten maximalen Farbtemperatur (K) auf der Strahlungslinie eines Schwarzen Körpers angepasst ist, (2) einer ersten LED-Korrektur-Lichtquelle mit einem Emissionsspektrum, dessen Maximum der Wellenlänge auf oder um den Schnittpunkt L1 (nm) liegt, der als Schnittpunkt der Verlängerung des Abschnitts der Geraden zwischen dieser minimalen Farbtemperatur Xmin und der Farbtemperatur des mittleren Punktes X mit der Hüllkurve des Chromatikdiagramms auf Seiten der größeren Wellenlängen bestimmt wird, sowie (3) einer zweiten LED-Korrekturlichtquelle mit einem Emissionsspektrum, dessen Maximum der Wellenlänge auf oder um den Schnittpunkt S1 (nm) liegt, der als Schnittpunkt der Verlängerung des Abschnitts der Geraden zwischen dieser maximalen Farbtemperatur Xmax und der Farbtemperatur des mittleren Punktes X mit der Hüllkurve des Chromatikdiagramms auf Seiten der geringeren Wellenlängen bestimmt wird, in welchem die resultierende Farbtemperatur des aus der weißen LED-Lichtquelle in Verbindung mit der ersten oder der zweiten LED-Korrekturlichtquelle erhaltenen Lichts mit Hilfe des Mischverhältnisses der Intensität der Spektren dieser beiden Lichtquellen so geregelt werden kann, dass eine Farbe erhalten wird, die an die minimale Farbtemperatur oder die maximale Farbtemperatur angenähert ist, sowie in welchem (1) die genannte weiße LED-Lichtquelle die Kombination aus einer LED mit einem Emissionsspektrum, dessen maximale Wellenlänge an den Bereich des blauen Licht von 430 bis 470 nm angepasst ist, sowie einem fluoreszierenden, von dieser LED aktivierten Stoff, dessen maximale Wellenlänge an den Bereich des gelben Licht von 530 bis 570 nm angepasst ist, darstellt und in welchem (2) die LED-Hilfs-Lichtquelle, die aus der oben erwähnten ersten und zweiten LED-Korrektur-Lichtquelle besteht, eine blaue LED, deren maximale Wellenlänge an den Bereich des blauen Licht von 430 bis 470 nm angepasst ist, sowie eine orangefarbene LED, deren maximale Wellenlänge an den Bereich des orangen Licht von 590 bis 630 nm angepasst ist, umfasst und in der die resultierende Farbtemperatur des aus einer beliebigen Lichtquelle unter der weißen LED-Lichtquelle und der LED-Hilfs-Lichtquelle bzw. der Verbindung dieser beiden Lichtquellen erzeugten Lichts im Bereich zwischen 3000 und 8000 K als Gesamtheit reguliert werden kann.

2. LED-Beleuchtungsanlage, bestehend aus (1) einer weißen LED-Lichtquelle, die an die zuvor festgelegte minimale Farbtemperatur Xlow (K) auf der Strahlungslinie eines Schwarzen Körpers angepasst ist, sowie (2) einer dritten LED-Korrekturlichtquelle mit einem Emissionsspektrum, dessen Maximum der Wellenlänge auf oder um den Schnittpunkt S2 (nm) liegt, der als Schnittpunkt der Verlängerung des Abschnitts der Geraden zwischen dieser minimalen Farbtemperatur Xlow und mindestens einer Farbtemperatur, die aus der Strahlungslinie eines Schwarzen Körpers auf der Seite der höheren Temperaturen ausgewählt wurde, mit der Hüllkurve des Chromatikdiagramms auf Seiten der geringeren Wellenlängen bestimmt wird, in welchem die resultierende Farbtemperatur des aus der weißen LED-Lichtquelle in Verbindung mit der dritten LED-Korrekturlichtquelle erhaltenen Lichts mit Hilfe des Mischverhältnisses der Intensität der Spektren dieser beiden Lichtquellen so geregelt werden kann, dass eine Farbe erhalten wird, die an die maximale Farbtemperatur angenähert ist, sowie in welchem (1) die genannte weiße LED-Lichtquelle die Kombination aus einer LED mit einer maximalen Wellenlänge des emittierten Lichts, die an den Bereich des blauen Licht von 430 bis 470 nm angepasst ist, sowie einem fluoreszierenden, von dieser LED aktivierten Stoff mit einer maximalen Wellenlänge der Fluoreszenz, die an den Bereich des gelben Licht von 530 bis 570 nm angepasst ist, darstellt und in welchem (2) die LED-Hilfs-Lichtquelle, die aus der oben erwähnten dritten LED-Korrektur-Lichtquelle besteht, eine blaue LED, deren maximale Wellenlänge des emittierten Lichts an den Bereich des blauen Licht von 430 bis 470 nm angepasst ist, sowie eine orangefarbene LED, deren maximale Wellenlänge an den Bereich des orangen Licht von 590 bis 630 nm angepasst ist, umfasst und in der die resultierende Farbtemperatur des aus einer beliebigen Lichtquelle unter der weißen LED-Lichtquelle und der LED-Hilfs-Lichtquelle bzw. der Verbindung dieser beiden Lichtquellen erzeugten Lichts im Bereich zwischen 3000 und 8000 K als Gesamtheit reguliert werden kann.

3. LED-Beleuchtungsanlage, bestehend aus (1) einer weißen LED-Lichtquelle, die an die zuvor festgelegte maximale Farbtemperatur Xhigh (K) auf der Strahlungslinie eines Schwarzen Körpers angepasst ist, sowie (2) einer vierten LED-Korrekturlichtquelle mit einem Emissionsspektrum, dessen Maximum der Wellenlänge auf oder um den Schnittpunkt L2 (nm) liegt, der als Schnittpunkt der Verlängerung des Abschnitts der Geraden zwischen dieser maximalen Farbtemperatur Xhigh und mindestens einer Farbtemperatur, die aus der Strahlungslinie eines Schwarzen Körpers auf der Seite der niedrigeren Temperaturen ausgewählt wurde, mit der Hüllkurve des Chromatikdiagramms auf Seiten der größeren Wellenlängen bestimmt wird, in welchem die resultierende Farbtemperatur des aus der weißen LED-Lichtquelle in Verbindung mit der vierten LED-Korrekturlichtquelle erhaltenen Lichts mit Hilfe des Mischverhältnisses der Intensität der Spektren dieser beiden Lichtquellen so geregelt werden kann, dass eine Farbe erhalten wird, die an die minimale Farbtemperatur angenähert ist, sowie in welchem (1) die genannte weiße LED-Lichtquelle die Kombination aus einer LED mit einer maximalen Wellenlänge des emittierten Lichts im Bereich des blauen Licht von 430 bis 470 nm sowie einem fluoreszierenden, von dieser LED aktivierten Stoff mit einer maximalen Wellenlänge der Fluoreszenz, die an den Bereich des gelben Licht von 530 bis 570 nm angepasst ist, darstellt und in welchem (2) die LED-Hilfs-Lichtquelle, die aus der oben erwähnten vierten LED-Korrektur-Lichtquelle besteht, eine blaue LED, deren maximale Wellenlänge der Emission an den Gesamtheit des blauen Licht von 430 bis 470 nm angepasst ist, sowie eine orangefarbene LED, deren maximale Wellenlänge der Emission an den Gesamtheit des orangen Licht von 590 bis 630 nm angepasst ist, umfasst und in der die resultierende Farbtemperatur des aus einer beliebigen Lichtquelle unter der weißen LED-Lichtquelle und der LED-Hilfs-Lichtquelle bzw. der Verbindung dieser beiden Lichtquellen erzeugten Lichts im Gesamtheit zwischen 3000 und 8000 K als Gesamtheit reguliert werden kann.

4. LED-Beleuchtungsanlage nach einem beliebigen unter den Patentansprüchen 1 bis 3, in welchem die genannte weiße LED-Lichtquelle ein Verhältnis der Intensität des Spektrums der durch ihre Schicht des fluoreszierenden Stoffes YAG durchlaufenden Strahlung zur Intensität des Emissionsspektrums der Schicht ihres fluoreszierenden Stoffes YAG im Bereich von 2:1 bis 1:4 aufweist.

5. LED-Beleuchtungsanlage nach einem beliebigen unter den Patentansprüchen 1 bis 4, die zusätzlich einen Anschluss für eine Wechselstromquelle, einen Transformator zur Änderung [der Parameter] der Wechselstromquelle und einen Lichtregler zum Anschluss des Transformators sowie zur Festlegung und Lieferung einer vorher festgelegten Gleichstromspannung entsprechend der weißen LED-Lichtquelle und der Hilfs-LED umfasst.

6. LED-Beleuchtungsanlage nach einem beliebigen unter den Patentansprüchen 1 bis 4, die zusätzlich einen Anschluss für eine Gleichstromquelle für die schattenfrei Beleuchtungsanlage, einen Transformator und einen Lichtregler zum Anschluss des Transformators sowie zur Festlegung und Lieferung einer vorher festgelegten Gleichstromspannung entsprechend der weißen LED-Lichtquelle und der Hilfs-LED umfasst.

7. LED-Beleuchtungsanlage nach einem beliebigen unter den Patentansprüchen 1 bis 4, in welchem die Grundfläche, an welcher die weiße LED-Lichtquelle und die zusätzlichen LED-Lichtquellen befestigt sind, von vorn mit einem sphärischen Reflexionsspiegel ausgestattet ist, um weißes Licht ohne farbliche Uneinheitlichkeiten als gespiegeltes Licht, das eine Farbmischung darstellt, zu erhalten.

8. LED-Beleuchtungsanlage nach einem beliebigen unter den Patentansprüchen 1 bis 4, die zusätzlich ein System zur Regulierung der Lichtmenge aus der LED-Hilfslichtquelle im Verhältnis zur weißen LED-Lichtquelle durch die Betätigung eines Schalters oder eines Fernsteuerungsmoduls (Fernbedienung) zum Zwecke einer solchen Regulierung enthält.

9. LED-Beleuchtungsanlage nach dem Patentansprüchen 8, in welcher das genannte System zur Regulierung der Lichtmenge aus der LED-Hilfslichtquelle im Verhältnis zur weißen LED-Lichtquelle durch die Betätigung eines Schalters oder eines Fernsteuerungsmoduls (Fernbedienung) zum Zwecke einer solchen Regulierung so konfiguriert ist, dass die Auswahl der optimalen Farbtemperatur für den Betriebsbereich möglich ist.

10. LED-Beleuchtungsanlage nach dem Patentansprüchen 8, in welcher das genannte System zur Regulierung der Lichtmenge aus der LED-Hilfslichtquelle im Verhältnis zur weißen LED-Lichtquelle durch die Betätigung eines Schalters oder eines Fernsteuerungsmoduls (Fernbedienung) zum Zwecke einer solchen Regulierung so konfiguriert ist, dass die Auswahl der Farbtemperatur für einen ausgewählten Bereich möglich ist.

11. LED-Beleuchtungsanlage nach dem Patentansprüchen 8, in welcher das genannte System zur Regulierung der Lichtmenge aus der LED-Hilfslichtquelle im Verhältnis zur weißen LED-Lichtquelle durch die Betätigung eines Schalters oder eines Fernsteuerungsmoduls (Fernbedienung) zum Zwecke einer solchen Regulierung so konfiguriert ist, dass die Auswahl der gewünschten, dem Tageslicht entsprechenden Farbtemperatur möglich ist.

12. Verfahren der Regulierung der Farbtemperatur des von der LED-Beleuchtungsanlage emittierten Lichts, in welcher:
(a) eine weißen LED-Lichtquelle, in welcher die Farbtemperatur des von dieser Quelle emittierten Lichts an die Farbtemperatur des mittleren Punktes X zwischen der zuvor festgelegten minimalen Farbtemperatur Xmin (K) und der zuvor festgelegten maximalen Farbtemperatur (K) auf der Strahlungslinie eines Schwarzen Körpers angepasst ist, (2) eine erste LED-Korrektur-Lichtquelle mit einem Emissionsspektrum, dessen Maximum der Wellenlänge auf oder um den Schnittpunkt L1 (nm) liegt, der als Schnittpunkt der Verlängerung des Abschnitts der Geraden zwischen dieser minimalen Farbtemperatur Xmin und der Farbtemperatur des mittleren Punktes X mit der Hüllkurve des Chromatikdiagramms auf Seiten der größeren Wellenlängen bestimmt wird, sowie (3) eine zweite LED-Korrektur-Lichtquelle mit einem Emissionsspektrum, dessen Maximum der Wellenlänge auf oder um den Schnittpunkt S1 (nm) liegt, der als Schnittpunkt der Verlängerung des Abschnitts der Geraden zwischen dieser minimalen Farbtemperatur Xmax und der Farbtemperatur des mittleren Punktes X mit der Hüllkurve des Chromatikdiagramms auf Seiten der geringeren Wellenlängen bestimmt wird, sicherstellt ist, oder
(b) eine weißen LED-Lichtquelle, die an die zuvor festgelegte minimale Farbtemperatur Xlow (K) auf der Strahlungslinie eines Schwarzen Körpers angepasst ist, sowie (2) eine dritte LED-Korrektur-Lichtquelle mit einem Emissionsspektrum, dessen Maximum der Wellenlänge auf oder um den Schnittpunkt S2 (nm) liegt, der als Schnittpunkt der Verlängerung des Abschnitts der Geraden zwischen dieser minimalen Farbtemperatur Xlow und der Farbtemperatur eines auf der Strahlungslinie des Schwarzen Körpers auf Seiten der höheren Farbtemperaturen ausgewählten Punktes X mit der Hüllkurve des Chromatikdiagramms auf Seiten der geringeren Wellenlängen bestimmt wird, sicherstellt ist, oder
(c) eine weißen LED-Lichtquelle, die an die zuvor festgelegte minimale Farbtemperatur Xhigh (K) auf der Strahlungslinie eines Schwarzen Körpers angepasst ist, sowie (2) eine vierte LED-Korrektur-Lichtquelle mit einem Emissionsspektrum, dessen Maximum der Wellenlänge auf oder um den Schnittpunkt S2 (nm) liegt, der als Schnittpunkt der Verlängerung des Abschnitts der Geraden zwischen dieser minimalen Farbtemperatur Xhigh und der Farbtemperatur eines auf der Strahlungslinie des Schwarzen Körpers auf Seiten der niedrigeren Farbtemperaturen ausgewählten Punktes X mit der Hüllkurve des Chromatikdiagramms auf Seiten der größeren Wellenlängen bestimmt wird, sicherstellt ist,
in welchem die resultierende Farbtemperatur das aus der weißen LED-Lichtquelle in Verbindung mit der LED-Korrekturlichtquelle mit Hilfe des Mischungsverhältnisses der Intensität der Spektren dieser beiden Lichtquellen so eingestellt wird, dass eine Farbe erhalten wird, die an die erwähnte minimale Farbtemperatur oder die erwähnte maximale Farbtemperatur angenähert ist.

13. Verfahren nach Patentanspruch 12, in welcher die LED-Beleuchtungsanlage eine Anlage nach einem der Patentansprüche 1 - 11 ist.

14. Verfahren nach Patentanspruch 13, in welchem die genannte weiße LED-Lichtquelle ein Verhältnis der Intensität des Spektrums der durch ihre Schicht des fluoreszierenden Stoffes YAG durchlaufenden Strahlung zur Intensität des Emissionsspektrums der Schicht ihres fluoreszierenden Stoffes YAG im Bereich von 2:1 bis 1:4 aufweist.

## Revendications

1. Le disposotif d'éclairage par DEL, contenant (1) une source DEL de la lumière blanche où la température de couleur de la lumière émise par cette source est adaptée à la température de la couleur du punktu moyen X entre la température minimum de la couleur Xmin (K) précédemment établie et la température maximum de la couleur (K) précédemment établie sur la ligne du rayonnement du corps noir, (2) la première source DEL corrigeante de la lumière possédant un spectre d'émission, pour lequel le maximum de la longeur de l'onde est situé sur ou autour du point d'intersection L1 (nm) établi en tant que point d'intersection du prolongement du segment de la droite entre cette température minimum de la couleur Xmin et la température de la couleur du point moyen X avec l'enveloppe du graphique chromatique au côté des longueurs plus longues des ondes, et (3) la deuxième source DEL corrigeante de la lumière possédant un spectre d'émission, pour lequel le maximum de la longeur de l'onde est situe sur ou autour du point d'intersection S1 (nm), établi en tant que point d'intersection du prolongement du segment de la droite entre la température maximum de la couleur Xmax et la température de la couleur du point moyen X avec l'enveloppe du graphique chromatique au côté des longueurs plus courtes des ondes ou la température résultante de la couleur de la lumière obtenue de la source DEL de la lumière blanche par rapport à la première ou à la deuxième source DEL corrigeante de la lumière peut être réglée à l'aide du rapport du mélange de l'intensité des deux sources de manière à obtenir la couleur qui s'approche à la température minimum de la couleur ou à la température maximum de la couleur, et ou (1) la source DEL de la lumière blanche est une combinaison d'une DEL possédant un spectre d'émission, dont le maximum de la longueur de l'onde est adapté à l'espace bleu de 430 à 470 nm et d'une substance fluorescente, incitée par la DEL et le maximum de la longueur de l'onde de sa fluorescence est adapté à l'espace jaune de 530 à 570 nm, et ou (2) la source DEL auxiliaire de la lumière contenant ladite première et deuxième source DEL corrigeante de la lumière contient la DEL bleue ou le maximum de la longuer de l'onde d'émission est adapté à l'espace bleu de 430 à 470 nm et la DEL où le maximum de la longuer de l'onde d'émission est adapte à l'espace orange de 590 à 630 nm et où la température résultante de la couleur de la lumière obtenue de n'importe quelle source DEL de la lumière blanche et de la source DEL auxiliaire ou de la liaison de ces deux sources de la lumière peut être réglée dans la plage de 3000 à 8000 K en tant qu'entité.

2. Le disposotif d'éclairage par DEL contenant (1) une source DEL de la lumière blanche adaptée à la température minimum de la couleur Xlow (K) précédemment établie à la ligne du rayonnement du corps noir, et (2) la troisième source DEL corrigeante de la lumière possédant un spectre d'émission, dont le maximum de la longueur de l'onde est situé sur ou autour du point d'intersection S2 (nm) établi en tant que point d'intersection du prolongement du segment de la droite entre la température minimum de la couleur Xlow et au moins une des températures de la couleur X choisie à la ligne du rayonnement du corps noir au côté des températures plus hautes avec l'enveloppe du graphique chromatique au côté des longueurs plus courtes des ondes ou la température résultante de la couleur de la lumière obtenue de la source DEL de la lumière blanche liée à la troisième source DEL corrigeante de la lumière peut être réglée à l'aide du rapport du mélange de l'intensité des spectres de ces deux lumières de manière à obtenir une couleur qui s'approche à la température maximum de la couleur et ou (1) ladite source DEL de la lumière blanche est une combinaison DEL ayant le maximum de la longueur de l'onde de la lumière émise adaptée à l'espace bleu de 430 à 470 nm et de la substance fluorescente, incitée par la DEL, ayant le maximum de la longueur de l'onde de sa fluorescence adapté à l'espace jaune de 530 à 570 nm, et ou (2) la source DEL auxiliaire de la lumière contenant ladite troisième source DEL corrigeante de la lumière contient une DEL bleue dont le maximum de la longueur de l'onde d'émission est adapté à l'espace bleu de 430 à 470 nm et une DEL orange, dont le maximum de la longueur de l'onde d'émission est adapte à l'espace orange de 590 à 630 nm et ou la température résultante de la couleur de la lumière obtenue de n'importe quelle source DEL de la lumière blanche ou de la source DEL auxiliaire de la lumière ou de la liaison de ces deux sources de la lumière peut être réglée dans la plage de 3000 à 8000 K en tant qu'entité.

3. Le dispositif d'éclairage par DEL contenant (1) une source DEL de la lumière blanche adaptée à la température maximum de la couleur Xhigh (K) précédemment établie à la ligne du rayonnement du corps noir, et (2) la quatrième source DEL corrigeante de la lumière possédant un spectre d'émission, dont le maximum de la longueur de l'onde est situé sur ou autour du point d'intersection L2 (nm), établi en tant que point d'intersection du prolongement du segment de la droite Xhigh et au moins une des températures de la couleur X choisie à la ligne du rayonnement du corps noir au côté des températures plus basses avec l'enveloppe du graphique chromatique au côté des longueurs plus longues des ondes ou la température résultante de la couleur de la lumière obtenue d'une source DEL de la lumière blanche liée à la quatrième source corrigeante DEL de la lumière peut être réglée à l'aide du rapport du mélange de l'intensité des spectres de ces deux lumières de manière à obtenir une couleur qui s'approche à la température maximum de la couleur et ou (1) ladite source DEL de la lumière blanche est une combinaison DEL ayant le maximum de la longueur de l'onde de la lumière émise adaptée à l'espace bleu de 430 à 470 nm et de la substance fluorescente, incitée par la DEL, ayant le maximum de la longueur de l'onde de sa fluorescence adaptée à l'espace jaune de 530 à 570 nm, et ou (2) la source DEL auxiliaire de la lumière contenant ladite quatrième source DEL corrigeante de la lumière contient une DEL bleue dont le maximum de la longueur de l'onde d'émission est adapté à l'espace bleu de 430 à 470 nm et une DEL orange, dont le maximum de la longueur de l'onde d'émission est adapté à l'espace orange de 590 à 630 nm et ou la température résultante de la couleur de la lumière obtenue de n'importe quelle source DEL de la lumière blanche ou de la source DEL auxiliaire de la lumière ou de la liaison de ces deux sources de la lumière peut être réglée dans la plage de 3000 à 8000 K en tant qu'entité.

4. Le dispositif d'éclairage par DEL selon n'importe quelle des revendications 1 à 3, où la source DEL de la lumière blanche a le rapport d'intensité du spectre du rayonnement passant par sa couche de la substance fluorescente YAG à l'intensité du spectre d'émission de la couche de sa substance fluorescente YAG contenu dans la plage de 2:1 à 1:4.

5. Le dispositif d'éclairage par DEL selon n'importe quelle des revendications 1 à 4, qui contient en plus la liasion de raccordement à la source du courant alternatif, le transformateur pour la modification [des caractéristiques] de la source du courant alternatif et le règlement de lumière pour raccorder au transformateur et établir et fournir la tension du courant continu précédemment établie, conformément à la source DEL de la lumière blanche et à la source DEL auxiliaire.

6. Le dispositif d'éclairage par DEL selon n'importe quelle des revendications 1 à 4, qui contient en plus la liaison de raccordement à la source du courant continu pour le dispositif d'éclairage sans ombre, le transformateur et le règlement de lumière pour raccorder au transformateur et établir et fournir la tension du courant continu précédemment établie, conformément à la source DEL de la lumière blanche et à la source DEL auxiliaire.

7. Le dispositif d'éclairage par DEL selon n'importe quelle des revendications 1 à 4, dont la base à laquelle les sources DEL de la lumière blanche et la source DEL complémentaire de la lumière sont fixées, est doté du front du miroir sphérique reflétant pour obtenir la lumière blanche sans irrrégularité chromatique, en tant que lumière reflétée étant un mélange des couleurs.

8. Le dispositif d'éclairage par DEL selon n'importe quelle des revendications. 1 à 4, qui contient en plus le système de règlement de la quantité de lumière de la source DEL auxiliaire de la lumière par rapport à la source DEL de la lumière blanche à travers la manipulation du commutateur ou le module de télécommande (télécommande) dans le but d'un tel règlement.

9. Le dispositif d'éclairage par DEL selon la revendication 8, ou le système de règlement de la quantité de lumière de la source DEL auxiliaire de la lumière par rapport à la source DEL de la lumière blanche à travers la manipulation du commutateur ou le module de télécommande (télécommande) dans le but d'un tel règlement est configuré de manière à rendre possible le choix de la température de la couleur optimum pour l'espace de l'opération.

10. Le dispositif d'éclairage par DEL selon la revendication 8, ou le système de règlement de la quantité de lumière de la source DEL auxiliaire de la lumière par rapport à la source DEL de la lumière blanche à travers la manipulation du commutateur ou le module de télécommande (télécommande) dans le but d'un tel règlement est configuré de manière à rendre possible le choix de la température de la couleur optimum pour l'espace choisi.

11. Le dispositif d'éclairage par DEL selon la revendication 8, ou le système de règlement de la quantité de lumière de la source DEL auxiliaire de la lumière par rapport à la source DEL de la lumière blanche à travers la manipulation du commutateur ou le module de télécommande (télécommande) dans le but d'un tel règlement est configuré de manière à rendre possible le choix de la température de la couleur propre à la lumière du jour.

12. La méthode du règlement de la température de la couleur de la lumière émise par le dispositif d'éclairage par DEL où:
(a) il est assuré une source DEL de la lumière blanche où la température de la lumière émise par cette source est adaptée à la température de la couleur du point moyen X entre la température minimum de la couleur Xmin (K) précédemment établie et la température maximum de la couleur (K) précédemment établie à la ligne du rayonnement du corps noir, (2) la première source DEL corrigeante de la lumière possédant un spectre d'émission dont le maximum de la longueur de l'onde est situé sur ou autour du point d'intersection L1 (nm) établi en tant que point d'intersection du prolongement du segment de la droite entre la température minimum de la couleur Xmin et la température de la couleur du point moyen X, avec l'enveloppe du graphique chromatique au côté des longueurs plus longues des ondes, et (3) la deuxième source DEL corrigeante de la lumière possédant un spectre d'émission dont le maximum de la longueur de l'onde est situé sur ou autour du point d'intersection S1 (nm), établi en tant que point d'intersection du segment de la droite entre entre la température maximum de la couleur Xmin et la température de la couleur du point moyen X avec l'enveloppe du graphique au côté des longueurs plus courtes des ondes, ou
(b) il est assuré une source DEL de la lumière blanche adaptée à la température minimum de la couleur Xlow (K) précédemment établie, à la ligne du rayonnement du corps noir, et (2) la troisième source DEL corrigeante de la lumière possédant un spectre d'émission dont le maximum de la longueur de l'onde est situé sur ou autour du point d'intersection S2 (nm) établi en tant que prolongement du segment de la droite entre la température minimum de la couleur Xlow et au moins une des températures de la couleur X choisie à la ligne du rayonnement du corps noir au côté des températures plus hautes avec l'enveloppe du graphique au côté des longueurs plus courtes des ondes, ou en plus
(c) il est assuré une source DEL de la lumière blanche adaptée à la température maximum de la couleur Xhigh (K) à la ligne du rayonnement du corps noir, et (2) la quatrième source DEL corrigeante de la lumière possédant un spectre d'émission dont le maximum de la longueur de l'onde est situé sur ou autour du point d'intersection L2 (nm), établi en tant que point d'intersection du prolongement du segment de la droite entre la température maximum Xhigh et au moins une des températures de la couleur X choisie à la ligne du rayonnement du corps noir au côté des températures plus basses, avec l'enveloppe du graphique chromatique au côté des longueurs plus longues des ondes,
où la température résultante de la couleur de la lumière obtenue de la source DEL de la lumière blanche liée à la source DEL corrigeante de la lumière est réglée à l'aide du rapport du mélange de l'intensité des spectres de ces deux sources de manière à obtenir une couleur qui s'approche à cette température minimum de la couleur ou à la température maximum de la couleur,

13. La méthode selon la revend. 12, où le dispositif d'éclairage par LED est un dispositif selon n'importe quelle revendication des revend. 1 -11.

14. La méthode selon la revend. 13, où la source DEL de la lumière blanche a le rapport de l'intensité du spectre du rayonnement passant par sa couche de la substance fluorescente YAG à l'intensité du spectre d'émission de la couche de sa substance fluorescente YAG contenu dans la plage de 2:1 à 1:4.
